# EUROPEAN PATENT SPECIFICATION

(11) **EP 3 574 945 B1**
(45) Date of publication and mention of the grant of the patent: **10.07.2024**
(21) Application number: 18305671.2
(22) Date of filing: 31.05.2018
(51) Int. Cl.: A61M 5/32

(54) **PROTECTIVE DEVICE COMPRISING A PROTECTIVE COVER WITH FIXED CLAWS**
SCHUTZVORRICHTUNG MIT EINER SCHUTZHÜLLE MIT FESTEN KLAUEN
DISPOSITIF DE PROTECTION COMPRENANT UN COUVERCLE DE PROTECTION À GRIFFES FIXES

(43) Date of publication of application: 04.12.2019
(73) Proprietor: Aspen Global Incorporated, Grand Bay (MU)
(72) Inventor: BLOQUEL, Fabrice, 76890 Varneville-Bretteville (FR)
(74) Representative: Novagraaf Technologies

(56) References cited:
- EP-A1- 2 913 076
- US-A1- 2003 181 859
- US-A1- 2012 191 046

## Description

The invention is directed at a protective device comprising a protective cover intended to be gripped on a needle-protecting cap of a syringe. Notably the invention relates to a protective cover having claws arranged in order to grip the said needle-protecting cap and being able to take the latter away upon the removal of the said cover.

It can for example apply to a syringe needle having a needle-protecting cap made of a flexible material and a protective cover made of a rigid material adapted to cover the needle-protecting.

Such protective devices are commonly used for safety reasons. They also facilitate the removal of the needle-protecting cap which is held within the protective cover upon the removal of the latter.

WO02/11799A1 discloses one example of these protective devices. In order to hold the needle-protecting cap, the protective cover is equipped with tilting claws. The object of these claws is to enable to fit the cover on the needle-protecting cap without pushing away said cap, as it may lead to damages to the end of the needle conventionally sticking into the cap. This design allows a lesser strength for fitting the cover on the needle-protecting cap. It also requires a stronger strength for removing the cover from this cap, which enables the withdrawal of the cover together with the cap, this latter being kept inside the cover by the claws.

However, once the protective device is mounted on a syringe, the cover being gripped on the needle-protecting cap, with time the articulation of the claws evolves because of creep of its material, especially with plastics. The result is that after a while the retaining force of the claws is lessen. In such a case, there is a risk that the protective cover may be removed without the cap, which stays on the needle.

The object of the present invention is therefore to provide with a protective device with an improved reliability in terms of life span.

To this end, the invention is directed at a protective device as defined in claim 1.

This enables to grip the said needle-protecting cap in order to take the cap away upon the removal of the said cover, without requiring a too strong force to fit the cover on the cap, even though the claws are without any articulation. And because these claws are fixed in respect of the internal surface, creep is less important. This postpone the time limit for using syringes with these protective device gripped on, compared to WO02/11799A1.

The invention can optionally comprise one or several features chosen among the following features:
- the protective cover comprises at least four of said claws ; this provides with a better retaining force ;
- the protective cover comprises only four or five of said claws ; this is a compromise between the force for forcing the needle-protecting cap inside the cover and the force retaining the needle-protecting cap in the cover;
- the protective device comprises only four of said claws ; this is the optimal compromise between the force for forcing the needle-protecting cap inside the cover and the force retaining the needle-protecting cap in the cover;
- the diameter of the circle passing through the point of the claws is equal or more than 3,4 mm and equal or less than 3,9 mm ;
- the diameter of the circle passing through the point of the claws is equal or more than 3,4 mm and equal or less than 3,8 mm ; better results are obtained with these values ; preferably, the diameter of the circle passing through the point of the claws is equal or more than 3,5 mm and equal or less than 3,7 mm ;
- said claws are distributed around a longitudinal axis of said cover;
- the claws are distributed approximatively symmetrically in respect of said longitudinal axis or in respect of a plane comprising said longitudinal axis ; in this paragraph approximatively means +/- 5°;
- in a plane perpendicular to said longitudinal axis and passing through the points of the claws, the angles between the points of adjacent claws are different, the smallest of these angles having a value that is equal or more than 70° and equal or less than 90°;
- in a plane perpendicular to said longitudinal axis and passing through the points of the claws, the angles between the points of adjacent claws are identical or approximatively identical ; in this paragraph approximatively means +/- 5°;
- for at least one of said claws, the intersection of this claw with the internal surface of said cover is the largest section of this corresponding claw ; the claw resistance to creep is even more enhanced;
- according to the invention, the claws are downstream oriented ; this decreases force for forcing the needle-protecting cap inside the cover and increases the retaining force that keeps the needle-protecting cap inside the cover;
- according to the invention, the claws have a point that narrows sharply compared to the rest of the claw ; the difference between the diameter of the circle passing through the end of the point of this claw and the diameter of the circle passing through the zone where the point of this claw narrows sharply is equal or more than 0,1 mm and equal or less than 0,2 mm ; this enables the point to better penetrate the material of the needle protecting cap ; it also avoids a shift between the upper and lower surface of the claw ;
- the protective device further comprises a needle protecting cap gripped by the claws and inside said cover; the protective device is ready for a syringe insertion;
- according to the invention, the cover is made of a rigid material and said cap is made of a flexible material ; this enables a better grip;
- when the protective device further comprises a needle protecting cap gripped by the claws and inside said cover, the protective device comprises a body for inserting a syringe, wherein the body has a separable portion delimited by a frangible zone, said separable portion constituting the protective cover; this enables to ensure that the protective device has not already been open.

The invention is also directed to an injection device comprising a protective device according to the invention, wherein a filled syringe is mounted inside said body, with a needle of this syringe being inserted inside said cap.

This injection device takes the benefits of the protective cover according to the invention.

Other features, objects and advantages of the invention will emerge from the following detailed description with reference to the attached drawings which show, by way of non-limiting example, a preferred embodiment thereof. In these drawings:
- Figure 1 is a longitudinal view of the injection device according to an embodiment of the invention and comprising the protective device according to an example of a first embodiment of the invention;
- Figure 2 is a longitudinal section of figure 1 on a longitudinal plane comprising the longitudinal axis A of the injection device ;
- Figure 3 is a bottom view of figure 1,
- Figure 4 is a zoom on a part IV of the longitudinal section of figure 2 but without the syringe;
- Figure 5 is a zoom on a part V of figure 3;
- Figures 6 to 8 are schematic cross-sections below the claws and according to a plane perpendicular to the longitudinal axis of a device according to the invention, these figures respectively corresponding to : an example of a second embodiment according to the invention, an example of a third embodiment according to the invention, the example of the first embodiment according to the invention;
- Figures 9 to 12 are schematic diagrams of examples of tests.

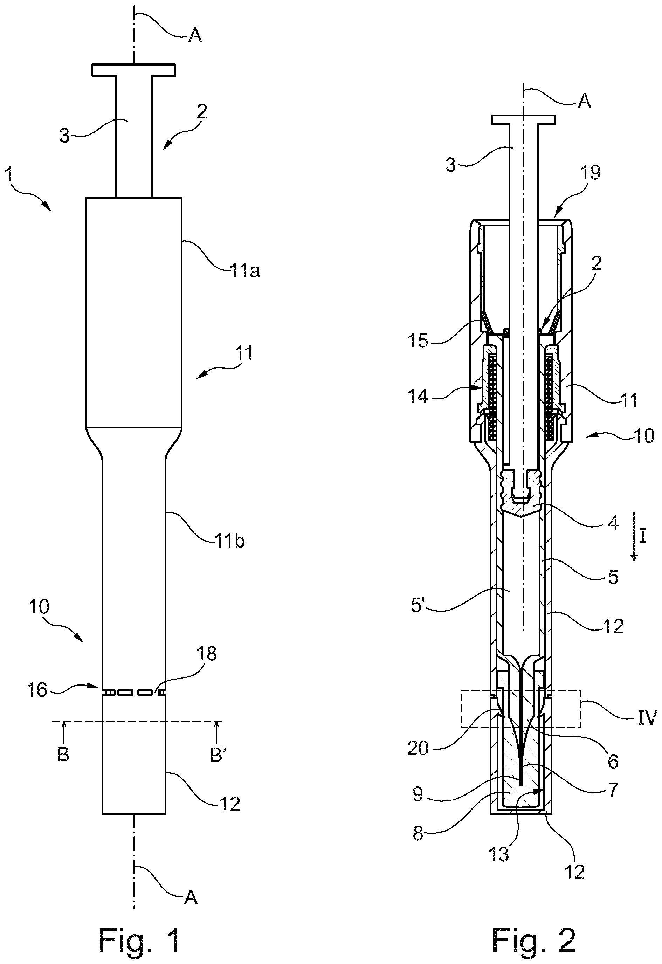

Figures 1 and 2 show an example of an injection device 1 according to the invention. This injection device can be, as in this example, a single-use injection device.

The injection device 1 comprises a protective device 10 and a syringe 2, here a prefilled syringe. The protective device 10 comprises a body, which here forms a protective case in which the syringe 2 is inserted and fixed.

The protective case comprises two case portions 11, 12. These latter can be tubular.

The first case portion 11 comprises in this example two tubular elements, front element 11b and rear element 11a, adapted to fit together as a continuation of one another, and to be preassembled before putting in place initially prefilled syringe 2 in the protective case 11, 12. However this first case portion 11 could be one single element made in a single block piece.

This first case portion is called here after the holding portion 11.

The second case portion 12 corresponds to the case 11 end and is made in one block piece with the front element 11b. However, this second case portion is here separable.

According to the invention, as in this example, this second case portion 12 is delimited by a frangible zone 16, which comprises holes and connecting frangible parts 18, these latter connecting the second case portion 12 to the front element 11b.

This second case portion 12 forms the protective end-piece of the protective device 10, that is to say the protective cover of the protective device 10. Said second case portion 12 is be called herein below the cover 12.

As mentioned above, the syringe 2 can be a traditional type of syringe, such as one made of glass for example, comprising:
- a tube 5 defining a product containing chamber 5',
- a piston 3 arranged inside the tube 5 so as to be able to slide toward the front, that is to say along direction I, in order to inject the product,
- a gasket 4 mounted on the piston 3 and sealing the rear of the said chamber 5',
- a nose 6 at the front of the tube 5,
- an injection needle 7 mounted on this nose 6,
- a needle-protecting cap 8 mounted on the nose 6 and having a cavity 9, in which the needle 7 is arranged.

Furthermore, as shown in figure 2, the protective device 10 is adapted so that when the syringe 2 is being put in place inside the protective device 10, the nose 6, the needle 7, and the needle-protecting cap 8 extend into the cover 12.

The cover 12 has an internal surface 13 and comprises internal claws protruding from this internal surface 13. The claws 20 are integral with the protective cover 12 and fixed in respect of the internal surface 13. The claws 20 can for example be integrally moulded with the cover 12.

In this first embodiment of the protective device 10, the cover 12 comprises four internal claws 20.

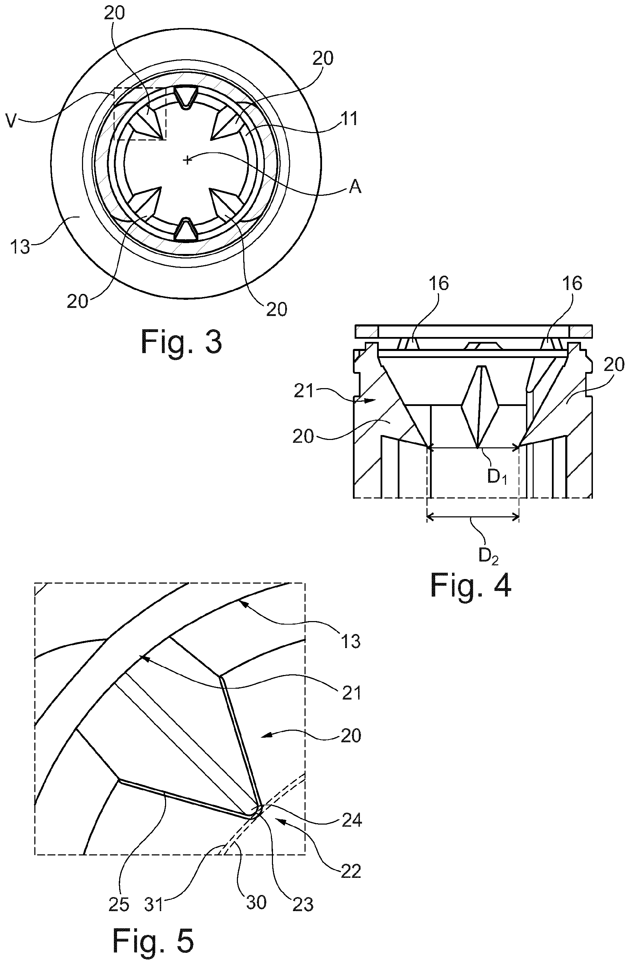

As can be seen on figures 3 to 5, each claw has a base 21, which corresponds to the junction of the claw 20 with the internal surface 13. Each claws 20 has also a free end in the form of a point 22.

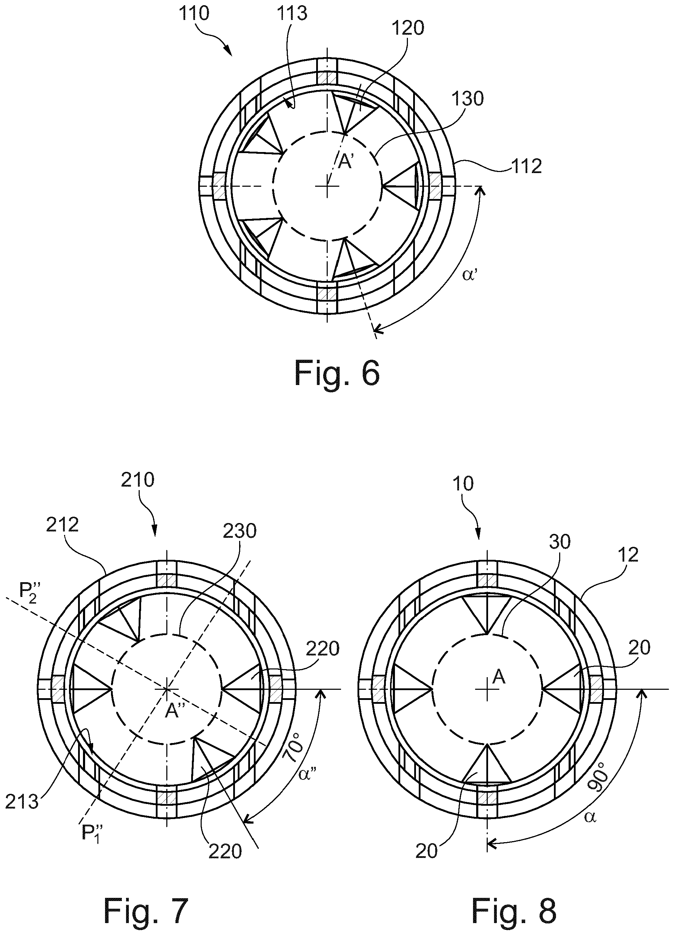

As shown on figure 8, each of the claw points 22 are arranged such as a circle 30 passes through theses points 22. This circle is circumscribed by these points 22 and is called herein below the first circumscribed circle 30.

The claws 20 are shaped so that the initial diameter D1 of the first circumscribed circle 30 is less than the diameter of the needle-protecting cap 8, so that it is necessary to force this cap 8 through the points 22 of the claws 20, when inserting the syringe 2 completely inside the protective case 11, 12. In other words, the protective device 10 is adapted to needle-protecting caps having a diameter higher than the initial diameter of the first circumscribed circle 30.

Therefore, when withdrawing the cover 12 from the holding part 11, the claws 20 penetrates into the needle-protecting cap 8. Therefore, cover 12 is gripped on the needle-protecting cap 8.

This enables to withdraw the needle-protecting cap 8 away from the nose 6 and needle 7 upon the removal of the cover 12.

The applicant has noted that the number of four claws 20 provides good resistance against a force applied in order to brake the connecting frangible parts 18 and separate the cover 12 from the holding portion 11, enabling the needle-protecting cap 8 staying inside the cover 12. Furthermore this number of claws, does not require a too strong force to fit the cover 12 on the cap 8.

Also, because these claws 20 are fixed in respect of the internal surface 13, creep is less important and provides the injection device 1 with an improved life span.

According to the invention, the diameter D1 of this first circumscribed circle 30 can be equal or more than 3,4 mm and equal or less than 3,9 mm. This improves the efficiency compromise between the force for forcing the needle-protecting cap 8 inside the cover 12 and the force retaining the needle-protecting cap 8 in the cover 12.

The efficiency compromise is improved when the diameter D1 of the first circumscribed circle 30 is equal or more than 3,4 mm and equal or less than 3,9 mm, even improved when it is equal or more than 3,4 mm and equal or less than 3,8 mm, and even improved when it is equal or more than 3,5 mm and equal or less than 3,7 mm. In the first embodiment example, this diameter D1 is about 3,6 mm.

In this first embodiment, claws 20 are uniformly distributed around the longitudinal axis A of said cover 12, which is also the longitudinal axis of the protective device 10 and of the injection device 1. Applicant has found that such distribution provides a better efficiency compromise.

This longitudinal axis A runs through the needle 7, once the syringe 2 is inserted in the protective case 11, 12, and is moreover aligned with the center of the first circumscribed circle 30.

To improve this efficiency compromise, the claws 20 can be downstream oriented, as can be seen in figure 4.

Here, the base 21 of each claws 20 has a section that is its largest section. Therefore, creep at the base 21 of each of the claws is lessen.

In this example, each of these claws 20 has the shape of a tetrahedron, the front face of which being visible in figures 3, 5 and 8.

As can be seen in figure 5, in this example, each claw 20 has a point 22 that narrows sharply compared to the rest of the claw 20. In this example, it can be seen that point 22 comprises a thick point portion 24 and a point extremity 23, which ends this point 22. Therefore, strength of the gripping is improved nearly without increasing the required strength for inserting the needle-protecting cap 8 in the cover 12. Indeed, the point extremity 23 penetrates more easily the cap 8, and ease further penetration of the thick point portion 24 in the cap 8 on insertion of the syringe 2.

The first circumscribed circle 30 passes at the free end of the point extremity 23. A second circumscribed circle 31 passes at the junction between the top of the thick point portion 24 and the point extremity 23, the claw narrowing sharply at this junction.

In this example, the difference between the diameter D2 of the second circumscribed circle 31 and the diameter D1 of the first circumscribed circle is equal or more than 0,1 mm and equal or less than 0,2 mm. Better results were obtained with these values.

As in this example, the cover 12 made of a rigid material and the needle-protecting cap 8 is made of a flexible material, enabling a better penetration and grip.

Mounting of the syringe 2 inside the protective device 10 is implemented as follows. The syringe 2 is inserted through rear opening 19 of the holding part 11 and slide from rear of the holding part 11 towards its end.

Upon this insertion, the needle-protecting cap 8 comes into contact with the points 22 of the claws 20 and is then forced through these claws. In this embodiment, only the material of this cap 8, and in some cases the point extremity 23 are deformed during this insertion.

When the prefilled syringe 2 is completely inserted, the injection device 1 is ready to use and can be stored. The connecting frangible parts 18 guarantee that the syringe 2 has not been used.

Subsequently, upon the removal of the protective cover 12, for the purpose of an injection, the connecting frangible parts 18 are broken and the claws 20 penetrates deeper inside the needle-protecting cap 8 and guarantees entrainment of the needle-protecting cap 8 away from the needle 7.

According to the invention, and as in this embodiment and shown in figure 2, the protective device 10 can comprise locking means 15 for rotational and translational locking of the syringe 2 inside this holding portion 11.

Also the protective device 10 can comprise elastic means 14, for example comprising a spring, which are able of bringing about an automatic withdrawal of the syringe 2 inside the protective case 11 at the end of the injection.

Examples of these locking means 15, elastic means 14 and automatic withdrawal of the syringe 2 are disclosed in FR2803530 A1.

The invention in general is not limited to the number of claws.

Improved results in terms of retaining force have been obtained with at least four claws.

In terms of compromise between the force for forcing the needle-protecting cap inside the cover and the force retaining the needle-protecting cap in the cover, the applicant has found that particular good results were obtained for a number of four or five claws.

The applicant has found that the best mode was with four claws 20 that are uniformly distributed around the longitudinal axis A, as in the first embodiment example.

The figure 6 represents a second embodiment with five claws 120 protruding from the internal face 113 of the protective cover 112.

For example, these claws 120 can be uniformly distributed around the longitudinal axis A' and a first circumscribed circle 130 can also be defined here, passing on claws 120 points.

Uniform distribution, in other words symmetric distribution of the claws in respect of said longitudinal axis, provides better results. That is to say, referring for example to first and the second embodiment and as can be seen respectively on figures 8 and 6, in a plane perpendicular to said longitudinal axis A, A' and passing through the points 22 of the claws 20, 120, the angles α, α' between the points of adjacent claws are identical or almost identical.

In a third embodiment, in figure 7, the claws 220 are not uniformly distributed. Good results were however obtained.

This results are improved when, as can be seen on figure 7, the claws 220 are distributed symmetrically in respect of a plane of symmetry P"1, P"2 comprising said longitudinal axis A".

In figure 7, the cover 212 has four claws 220 that are arranged such that two plan of symmetry P"1, P"2 can be defined: the first plan P"1 passing in the middle of the of the biggest angles between the points of adjacent claws, and the second plan P"2 passing in the middle of the smallest angles between the points of adjacent claws.

Results are further improved when, in a plane perpendicular to said longitudinal axis A" and passing through the points of the claws 220, the angles between the points of adjacent claws being different, the smallest angle α" among these angles has a value that is equal or more than 70° and equal or less than 90°. Here this value is 70°.

In order to illustrate the improvements of the different values, tests have been conducted with syringes that have a tube of 80,7 millimeters long.

Forces have been applied to the protective device 10 along the longitudinal axis A, A' or A":
- Insertion force is the force required to fully insert the needle-protecting cap 8 inside the cover 12,
- Gripping force is the force required to withdraw the needle-protecting cap 8 from the cover 12, after being fully inserted.

Good results are considered to be obtained when insertion force is lower than 15 Newtons (N) and gripping force is higher than 25 N.

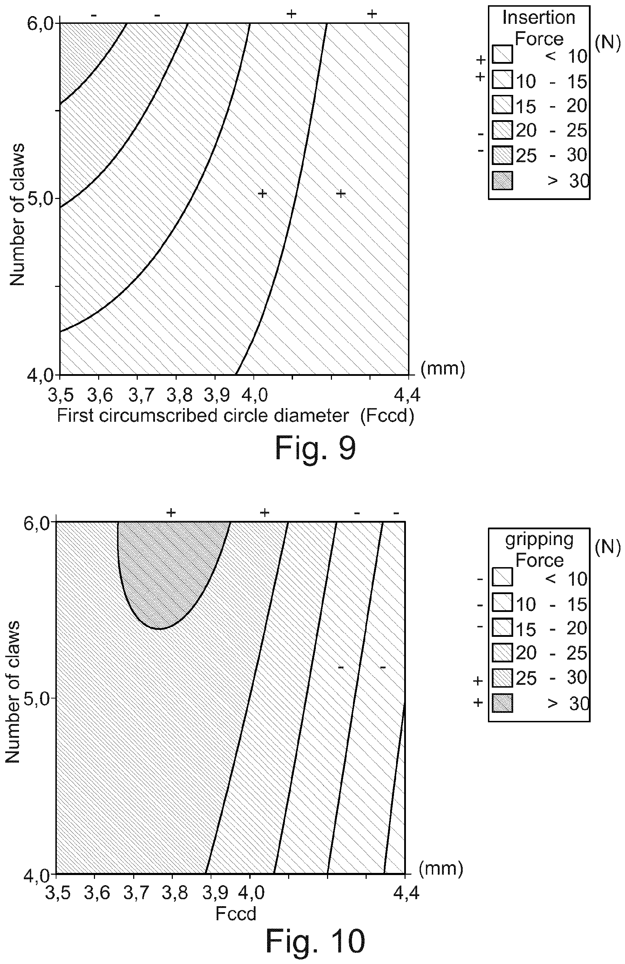

Figures 9 and 10 schematic diagrams are showing force values in respect of the number of claws and the first circumscribed circle 30 diameter D1 (Feed). Zones with (+) have good results, zones with (-) have bad results. Zones with no sign are intermediate zones.

These diagrams show that improved results are obtained with four and five claws and/or an Feed comprised between 3,4 and 3,9 mm.

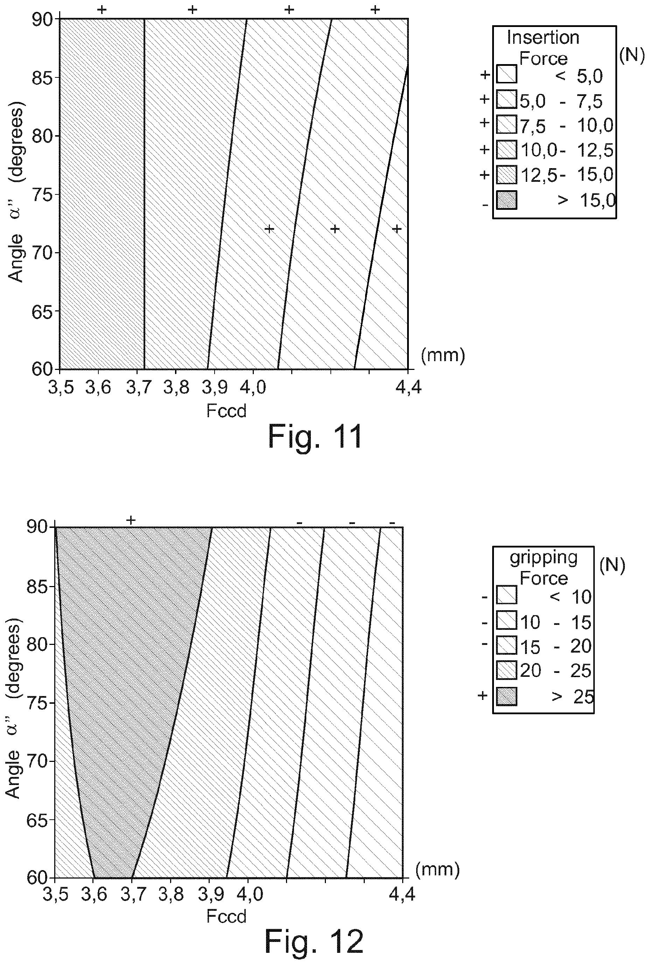

Figures 11 and 12 relates to an embodiment with four claws and are schematic diagrams showing force values in respect of the smallest angle α, α" between adjacent claws and the Feed. Zones with (+) have good results, zones with (-) have acceptable but less good results. Zones with no sign are intermediate zones.

These diagrams show that improved results are obtained with:
- an angle α, α" comprised between 60 and 90°, more preferably between 70 and 90°, and/or
- an Feed comprised between 3,4 and 3,9 mm.

Better results are obtained for an Feed comprised between 3,5 and 3,7 mm and more particularly 3,6 mm.

These diagrams also show that the best compromise is with:
- four claws 20,
- an angle α of 90°, which means a uniform distribution of the four claws around the longitudinal axis A, and
- an Feed of 3,6 mm.

## Claims

1. Protective device (10; 110; 210) comprising a protective cover (12) and further comprising a needle-protecting cap (8) inside the protective cover (12), wherein the protective cover (12) is made of a rigid material, intended to be gripped on the needle-protecting cap (8), made of a flexible material, of a syringe (2) to take the needle-protecting cap (8) away from the needle upon the removal of the said protective cover (12), said protective cover (12) having an internal surface (13; 113; 213) and comprising claws (20; 120; 220) protruding from this internal surface, said claws (20; 120; 220) configured to grip the needle-protecting cap (8) and the claws (20; 120; 220) having a free end in the form of a point (22) arranged so as to stick into the needle-protecting cap (8), wherein the claws (20; 120; 220):
- are downstream oriented,
- are integral with the protective cover (12),
- and **characterised in that** the claws (20; 120; 220) are fixed in respect of said internal surface (13; 113; 213) without any articulation.

2. Protective device (10; 110; 210) according to claim 1, wherein the protective cover (12) comprises only four or only five of said claws (20; 120; 220).

3. Protective device (10; 110; 210) according to claim 2, comprising only four of said claws (20; 120; 220).

4. Protective device (10; 110; 210) according to any of preceding claims, wherein the diameter of the circle (30) passing through the point (22) of the claws is equal or more than 3,4 mm and equal or less than 3,9 mm.

5. Protective device (10; 110; 210) according to claim 4, wherein the diameter of the circle (30; 130; 230) passing through the point (22) of the claws is equal or more than 3,4 mm and equal or less than 3,8 mm.

6. Protective device (10; 110; 210) according to claim 5, wherein the diameter of the circle (30; 130; 230) passing through the point (22) of the claws (20; 120; 220) is equal or more than 3,5 mm and equal or less than 3,7 mm.

7. Protective device (10; 110; 210) according to any of preceding claims, wherein said claws (20; 120; 220) are distributed around a longitudinal axis (A) of said cover (12).

8. Protective device (10; 110; 210) according to claim 7, wherein the claws (20; 120; 220) are distributed approximatively symmetrically in respect of said longitudinal axis (A; A') and/or in respect of a plane (P"1, P"2) comprising said longitudinal axis.

9. Protective device (10; 110; 210) according to claim 7 or according to claim 8, wherein in a plane perpendicular to said longitudinal axis (A") and passing through the points (22) of the claws (220), the angles between the points of adjacent claws are different, the smallest of these angles (α") having a value that is equal or more than 70° and equal or less than 90°.

10. Protective device (10; 110; 210) according to any of the preceding claims, wherein for at least one of said claws (20; 120; 220), the intersection of this claw with the internal surface (13; 113; 213) of said cover (12; 112; 212) is the largest section of this corresponding claw.

11. Protective device (10; 110; 210) according to any of the preceding claims, wherein at least one of the claws (20; 120; 220) has a point (22) that narrows sharply compared to the rest of the claw.

12. Protective device (10; 110; 210) according to claim 1, comprising a body (11, 12) for inserting a syringe (2), wherein the body (11, 12) has a separable portion delimited by a frangible zone (6), said separable portion constituting the protective cover (12; 112; 212).

13. Injection device (1) comprising a protective device (10; 110; 210) according to claim 12, wherein a filled syringe (2) is mounted inside said body (11, 12), with a needle (7) of this syringe being inserted inside said needle-protecting cap (8).

## Patentansprüche

1. Schutzvorrichtung (10; 110; 210) umfassend eine Schutzhülle (12) und ferner umfassend
eine Nadelschutzkappe (8) innerhalb der Schutzhülle (12), wobei die Schutzhülle (12) aus einem starren Material hergestellt ist,
die vorgesehen ist, um an der Nadelschutzkappe (8), die aus einem flexiblen Material hergestellt ist, einer Spritze (2) gegriffen zu werden, um bei der Entfernung der Schutzhülle (12) die Nadelschutzkappe (8) von der Nadel weg zu nehmen, die Schutzhülle (12) aufweisend eine Innenoberfläche (13; 113; 213) und umfassend Klauen (20; 120; 220), die von dieser Innenoberfläche vorstehen, wobei die Klauen (20; 120; 220) konfiguriert sind, um die Nadelschutzkappe (8) zu greifen und die Klauen (20; 120; 220) ein freies Ende in der Form einer Spitze (22) aufweisen, das angeordnet ist, um in die Nadelschutzkappe (8) hineinzuragen, wobei die Klauen (20; 120; 220):
- nachgelagert orientiert sind,
- mit der Schutzhülle (12) einstückig sind,
- und **dadurch gekennzeichnet, dass** die Klauen (20; 120; 220) in Bezug auf die Innenoberfläche (13; 113; 213) ohne jegliche Gelenkverbindung befestigt sind.

2. Schutzvorrichtung (10; 110; 210) nach Anspruch 1, wobei die Schutzhülle (12) nur vier oder nur fünf der Klauen (20; 120; 220) umfasst.

3. Schutzvorrichtung (10; 110; 210) nach Anspruch 2, umfassend nur vier der Klauen (20; 120; 220).

4. Schutzvorrichtung (10; 110; 210) nach einem der vorstehenden Ansprüche, wobei der Durchmesser des Kreises (30), der durch die Spitze (22) der Klauen hindurchgeht, gleich oder mehr als 3,4 mm und gleich oder weniger als 3,9 mm ist.

5. Schutzvorrichtung (10; 110; 210) nach Anspruch 4, wobei der Durchmesser des Kreises (30; 130; 230), der durch die Spitze (22) der Klauen hindurchgeht, gleich oder mehr als 3,4 mm und gleich oder weniger als 3,8 mm ist.

6. Schutzvorrichtung (10; 110; 210) nach Anspruch 5, wobei der Durchmesser des Kreises (30; 130; 230), der durch die Spitze (22) der Klauen (20; 120; 220) hindurchgeht, gleich oder mehr als 3,5 mm und gleich oder weniger als 3,7 mm ist.

7. Schutzvorrichtung (10; 110; 210) nach einem der vorstehenden Ansprüche, wobei die Klauen (20; 120; 220) um eine Längsachse (A) der Hülle (12) herum verteilt sind.

8. Schutzvorrichtung (10; 110; 210) nach Anspruch 7, wobei die Klauen (20; 120; 220) in Bezug auf die Längsachse (A; A') und/oder in Bezug auf eine Ebene (P"1, P"2), umfassend die Längsachse, approximativ symmetrisch verteilt sind.

9. Schutzvorrichtung (10; 110; 210) nach Anspruch 7 oder 8, wobei in einer Ebene, die senkrecht zu der Längsachse (A") ist und durch die Spitzen (22) der Klauen (220) hindurchgeht, die Winkel zwischen den Spitzen angrenzender Klauen unterschiedlich sind, wobei der kleinste dieser Winkel (α") einen Wert aufweist, der gleich oder mehr als 70° und gleich oder weniger als 90° ist.

10. Schutzvorrichtung (10; 110; 210) nach einem der vorstehenden Ansprüche, wobei für mindestens eine der Klauen (20; 120; 220) der Schnittpunkt dieser Klaue mit der Innenoberfläche (13; 113; 213) der Hülle (12; 112; 212) der größte Abschnitt dieser entsprechenden Klaue ist.

11. Schutzvorrichtung (10; 110; 210) nach einem der vorstehenden Ansprüche, wobei mindestens eine der Klauen (20; 120; 220) eine Spitze (22) aufweist, die sich im Vergleich zu dem Rest der Klaue stark verjüngt.

12. Schutzvorrichtung (10; 110; 210) nach Anspruch 1, umfassend einen Körper (11, 12) zum Einführen einer Spritze (2), wobei der Körper (11, 12) einen abtrennbaren Teil aufweist, der mittels einer zerbrechlichen Zone (6) begrenzt ist, wobei der abtrennbare Teil die Schutzhülle (12; 112; 212) bildet.

13. Injektionsvorrichtung (1) umfassend eine Schutzvorrichtung (10; 110; 210) nach Anspruch 12,
wobei eine gefüllte Spritze (2) innerhalb des Körpers (11, 12) montiert ist, wobei eine Nadel (7) dieser Spritze innerhalb der Nadelschutzkappe (8) eingeführt ist.

## Revendications

1. Dispositif de protection (10 ; 110 ; 210) comprenant un couvercle de protection (12) et comprenant en outre
un capuchon de protection d'aiguille (8) à l'intérieur du couvercle de protection (12), dans lequel le couvercle de protection (12) est constitué d'un matériau rigide,
destiné à être saisi sur le capuchon de protection d'aiguille (8), constitué d'un matériau souple, d'une seringue (2) pour éloigner le capuchon de protection d'aiguille (8) de l'aiguille lors du retrait dudit capuchon de protection (12), ledit capuchon de protection (12) ayant une surface interne (13 ; 113 ; 213) et comprenant des griffes (20 ; 120 ; 220) faisant saillie à partir de cette surface interne, lesdites griffes (20 ; 120 ; 220) conçues pour saisir le capuchon de protection d'aiguille (8) et les griffes (20 ; 120 ; 220) ayant une extrémité libre en forme d'une pointe (22) disposée de manière à s'enfoncer dans le capuchon de protection d'aiguille (8), dans lequel les griffes (20 ; 120 ; 220) :
- sont orientées vers l'aval,
- sont partie intégrante du couvercle de protection (12),
- et **caractérisé en ce que** les griffes (20 ; 120 ; 220) sont fixées par rapport à ladite surface interne (13 ; 113 ; 213) sans aucune articulation,

2. Dispositif de protection (10 ; 110 ; 210) selon la revendication 1, dans lequel le couvercle de protection (12) ne comprend que quatre ou cinq desdites griffes (20 ; 120 ; 220).

3. Dispositif de protection (10 ; 110 ; 210) selon la revendication 2, comprenant seulement quatre desdites griffes (20 ; 120 ; 220).

4. Dispositif de protection (10 ; 110 ; 210) selon l'une quelconque des revendications précédentes, dans lequel le diamètre du cercle (30) passant par la pointe (22) des griffes est supérieur ou égal à 3,4 mm et inférieur ou égal à 3,9 mm.

5. Dispositif de protection (10 ; 110 ; 210) selon la revendication 4, dans lequel le diamètre du cercle (30 ; 130 ; 230) passant par la pointe (22) des griffes est supérieur ou égal à 3,4 mm et inférieur ou égal à 3,8 mm.

6. Dispositif de protection (10 ; 110 ; 210) selon la revendication 5, dans lequel le diamètre du cercle (30 ; 130 ; 230) passant par la pointe (22) des griffes (20 ; 120 ; 220) est supérieur ou égal à 3,5 mm et inférieur ou égal à 3,7 mm.

7. Dispositif de protection (10 ; 110 ; 210) selon l'une quelconque des revendications précédentes, dans lequel lesdites griffes (20 ; 120 ; 220) sont réparties autour d'un axe longitudinal (A) dudit couvercle (12).

8. Dispositif de protection (10 ; 110 ; 210) selon la revendication 7, dans lequel les griffes (20 ; 120 ; 220) sont réparties de manière approximativement symétrique par rapport audit axe longitudinal (A ; A') et/ou par rapport à un plan (P "1, P "2) comprenant ledit axe longitudinal.

9. Dispositif de protection (10 ; 110 ; 210) selon la revendication 7 ou selon la revendication 8, dans lequel, dans un plan perpendiculaire audit axe longitudinal (A") et passant par les pointes (22) des griffes (220), les angles entre les pointes des griffes adjacentes sont différents, le plus petit de ces angles (α") ayant une valeur qui est égale ou supérieure à 70° et égale ou inférieure à 90°.

10. Dispositif de protection (10 ; 110 ; 210) selon l'une quelconque des revendications précédentes, dans lequel pour au moins une desdites griffes (20 ; 120 ; 220), l'intersection de cette griffe avec la surface interne (13 ; 113 ; 213) dudit couvercle (12 ; 112 ; 212) est la plus grande section de cette griffe correspondante.

11. Dispositif de protection (10 ; 110 ; 210) selon l'une quelconque des revendications précédentes, dans lequel au moins une des griffes (20 ; 120 ; 220) possède une pointe (22) qui se rétrécit fortement par rapport au reste de la griffe.

12. Dispositif de protection (10 ; 110 ; 210) selon la revendication 1, comprenant un corps (11, 12) pour l'insertion d'une seringue (2), dans lequel le corps (11, 12) a une partie séparable délimitée par une zone frangible (6), ladite partie séparable constituant le couvercle de protection (12 ; 112 ; 212).

13. Dispositif d'injection (1) comprenant un dispositif de protection (10 ; 110 ; 210) selon la revendication 12,
dans lequel une seringue remplie (2) est montée à l'intérieur dudit corps (11,12), une aiguille (7) de cette seringue étant insérée à l'intérieur dudit capuchon de protection d'aiguille (8).
